# EUROPEAN PATENT APPLICATION

(11) **EP 4 640 217 A1**
(43) Date of publication of application: **29.10.2025**
(21) Application number: 23907106.1
(22) Date of filing: 20.12.2023
(51) Int. Cl.: A61K 31/198, A61P 35/00

(54) **ANTICANCER AGENT**

(30) Priority: 20.12.2022 JP 2022203332; 10.11.2023 JP 2023192426
(71) Applicant: AGC INC., Chiyoda-ku, Tokyo 1008405 (JP); The University of Tokyo, Bunkyo-ku, Tokyo 113-8654 (JP)
(72) Inventor: OKAZOE Takashi, Tokyo 100-8405 (JP); KASHIWAGI Kimiaki, Tokyo 100-8405 (JP); OTA Yu, Tokyo 100-8405 (JP); OSAWA Tsuyoshi, Tokyo 113-8654 (JP); AIKAWA Kohsuke, Tokyo 113-8654 (JP); AKI Sho, Tokyo 113-8654 (JP); SUGAYA Maki, Tokyo 113-8654 (JP)
(74) Representative: Müller-Boré & Partner Patentanwälte PartG mbB
(86) International application number: PCT/JP2023/045787
(87) International publication number: WO 2024/135746

(57) **Abstract**

The present invention provides an anticancer agent having excellent cell membrane permeability and a pharmaceutical composition containing the anticancer agent. Namely, the present invention provides an anticancer agent containing, as an active ingredient, a fluorine-containing amino acid in which at least one hydrogen atom bonded to a carbon atom of an α-amino acid has been substituted with a fluorine atom, or a pharmacologically acceptable salt thereof; and also provides the anticancer agent described above in which the fluorine-containing amino acid has a monofluoromethyl group, a difluoromethyl group, or a trifluoromethyl group; and a pharmaceutical composition containing either of the above anticancer agents.

## Description

### TECHNICAL FIELD

The present invention relates to a novel anticancer agent and a pharmaceutical composition containing the anticancer agent.

Priority is claimed on Japanese Patent Application No. 2022-203332, filed December 20, 2022 and Japanese Patent Application No. 2023-192426, filed November 10, 2023, the contents of which are incorporated herein by reference.

### BACKGROUND ART

Fluorine-containing amino acids have been reported as exhibiting unique bioactivity, and are garnering much attention. For example, 3,3,3-trifluoroalanine and derivatives thereof have been reported as functioning as suicide inhibitors of pyridoxal enzymes (Non-Patent Document 1). Further, it has also been reported that alanine racemase from the Gram-negative bacterium Salmonella typhimurium and the Gram-positive bacterium Bacillus stearothermophilus are inactivated by 3,3,3-trifluoroalanine (Non-Patent Document 2). Fluorine-containing amino acids and peptides containing these amino acids hold much promise for use as bioactive substances in the pharmaceutical field.

In pharmaceuticals, ensuring that the pharmacologically active substance reaches the target molecule located inside a target cell is very important. Known drug delivery agents for pharmacologically active substances include lipid nanoparticles (Patent Document 1) and cationic polymer nanoparticles (Patent Document 2).

On the other hand, compounds having a polyfluoro structure are known to be stable in vivo, have low toxicity, and exhibit excellent intracellular uptake and excellent endosomal escape (Non-Patent Document 3).

### Citation List

### Patent Document

Patent Document 1: International Patent Publication No. 2011/036557
Patent Document 2: International Patent Publication No. 2017/212006

### Non Patent Documents

Non Patent Document 1: Sakai et al., Tetrahedron, 1996, vol. 52(1), pp. 233 to 244.
Non Patent Document 2: Faraci and Walsh, Biochemistry, 1989, vol. 28(2), pp. 431 to 437.
Non Patent Document 3: Zhang et al., MRS Communications, 2018, vol. 8, pp. 303 to 313.
Non Patent Document 4: Nodwell et al., Journal of the American Chemical Society, 2017, vol. 139, pp. 3595 to 3598.

### SUMMARY OF INVENTION

### Technical Problem

The present invention has an object of providing a novel anticancer agent and a pharmaceutical composition containing the anticancer agent.

### Solution to Problem

The inventors of the present invention discovered that a fluorine-containing amino acid in which at least one hydrogen atom bonded to a carbon atom within the side chain of an α-amino acid has been substituted with a fluorine atom exhibits excellent cell membrane permeability and a superior proliferation inhibitory action against cancer cells, thus enabling them to complete the present invention.

In other words, the present invention includes the following aspects.
[1] An anticancer agent containing, as an active ingredient, a fluorine-containing amino acid in which at least one hydrogen atom bonded to a carbon atom of an α-amino acid has been substituted with a fluorine atom, or a pharmacologically acceptable salt thereof.
[2] The anticancer agent according to [1], wherein the fluorine-containing amino acid has a monofluoromethyl group, a difluoromethyl group, or a trifluoromethyl group.
[3] The anticancer agent according to [1] or [2], wherein the α-amino acid is at least one amino acid selected from the group consisting of valine, leucine, isoleucine and glutamine.
[4] The anticancer agent according to [1], wherein the fluorine-containing amino acid is at least one amino acid selected from the group consisting of monofluoroleucine, trifluoroleucine, monofluorovaline, hexafluorovaline, monofluoroisoleucine, monofluoroglutamine and difluoroglutamine.
[5] A pharmaceutical composition containing the anticancer agent according to any one of [1] to [4].

### Advantageous Effects of Invention

The anticancer agent according to the present invention is a superior anticancer agent which exhibits excellent cell membrane permeability, a superior proliferation inhibitory action against cancer cells, and ready uptake into target cancer cells.

### DESCRIPTION OF EMBODIMENTS

In the present invention and in this description, the term "halogen atom" refers to a fluorine atom, a chlorine atom, a bromine atom, or an iodine atom. The expression "halogen atom other than a fluorine atom" means a chlorine atom, a bromine atom, or an iodine atom. The "halogen atom other than a fluorine atom" is preferably a chlorine atom or a bromine atom, and a chlorine atom is particularly desirable.

In the present invention and in this description, the abbreviation "Cₚ₁₋ₚ₂" (wherein p1 and p2 are positive integers that satisfy p1<p2) means a group having p1 to p2 carbon atoms.

In the present invention and in this description, a "C₁₋₆ alkyl group" is an alkyl group having 1 to 6 carbon atoms, and may have either a linear chain or a branched chain. Examples of the C₁₋₆ alkyl group include a methyl group, ethyl group, propyl group, isopropyl group, butyl group, isobutyl group, sec-butyl group, tert-butyl group, pentyl group, isopentyl group, neopentyl group, tert-pentyl group, and hexyl group.

The anticancer agent according to the present invention has, as an active ingredient, a fluorine-containing amino acid in which at least one hydrogen atom bonded to a carbon atom of an α-amino acid has been substituted with a fluorine atom, or a pharmacologically acceptable salt thereof. Because the fluorine-containing amino acid has a fluorine atom, the cell membrane permeability is excellent. As a result, the anticancer agent according to the present invention can be introduced into a target cancer cell simply by bringing the anticancer agent into contact with the surface of the cancer cell. Further, the fluorine-containing amino acid has a powerful proliferation inhibitory action against cancer cells, and therefore also has an excellent antitumor effect. The fluorine-containing amino acid also exhibits a proliferation inhibitory action on normal cells, but that action is weaker than that exhibited on cancer cells.

The fluorine-containing amino acid that functions as an active ingredient of the anticancer agent according to the present invention may be any compound in which at least one hydrogen atom bonded to a carbon atom of an α-amino acid has been substituted with a fluorine atom, but a compound in which at least one hydrogen atom bonded to a carbon atom that constitutes the side chain has been substituted with a fluorine atom is preferred, a fluorine-containing amino acid in which 1 to 6 hydrogen atoms each bonded to a carbon atom constituting the side chain have each been substituted with a fluorine atom is more preferred, and a fluorine-containing amino acid having one or two monofluoromethyl groups, difluoromethyl groups or trifluoromethyl groups is even more preferred.

The fluorine-containing amino acid that functions as an active ingredient of the anticancer agent according to the present invention is preferably a fluorine-containing amino acid in which at least one hydrogen atom bonded to a carbon atom of at least one α-amino acid selected from the group consisting of valine, leucine and isoleucine (hereinafter sometimes referred to as "branched-chain α-amino acids") has been substituted with a fluorine atom. The fluorine-containing amino acid that functions as an active ingredient of the anticancer agent according to the present invention is preferably a fluorine-containing amino acid in which 1 to 6 hydrogen atoms within the side chain of a branched-chain α-amino acid have each been substituted with a fluorine atom, and is more preferably a fluorine-containing amino acid having one or two monofluoromethyl groups, difluoromethyl groups or trifluoromethyl groups. The side chains of valine, leucine and isoleucine are an isopropyl group, an isobutyl group and a sec-butyl group respectively, each of which is bonded to the α-carbon atom. The fluorine-containing amino acid that functions as an active ingredient of the anticancer agent according to the present invention is particularly preferably at least one amino acid selected from the group consisting of monofluoroleucine (CAS RN: 857026-04-1, 4-fluoro-L-leucine), trifluoroleucine (CAS RN: 2260931-25-5, 2-amino-5,5,5-trifluoro-4-methylpentanoic acid hydrochloride), monofluorovaline (CAS RN: 43163-94-6, 3-fluoro-L-valine), hexafluorovaline (CAS RN: 16063-80-2, 4,4,4,4',4',4'-hexafluoro-DL-valine), monofluoroisoleucine (3-fluoroisoleucine (CAS RN: 79205-62-2, 3-fluoroisoleucine)), and 4-fluoroisoleucine (CAS RN: 2643315-39-1, 4-fluoro-isoleucine).

The fluorine-containing amino acid that functions as an active ingredient of the anticancer agent according to the present invention is also preferably an α-amino acid in which the side chain is linear. The fluorine-containing amino acid that functions as an active ingredient of the anticancer agent according to the present invention is preferably a fluorine-containing amino acid in which 1 to 6 hydrogen atoms within the side chain of an α-amino acid having a linear side chain have each been substituted with a fluorine atom, and is more preferably a fluorine-containing amino acid having one or two monofluoromethyl groups, difluoromethyl groups or trifluoromethyl groups. A fluorine-containing amino acid in which at least one hydrogen atom bonded to a carbon atom of glutamine has been substituted with a fluorine atom is also preferred as the fluorine-containing amino acid that functions as an active ingredient of the anticancer agent according to the present invention, and monofluoroglutamine in which one hydrogen atom bonded to a carbon atom of glutamine has been substituted with a fluorine atom, and difluoroglutamine in which two hydrogen atoms bonded to a carbon atom of glutamine have each been substituted with a fluorine atom are particularly desirable.

In the fluorine-containing amino acid that functions as an active ingredient of the anticancer agent according to the present invention, a hydrogen atom that has not been substituted with a fluorine atom may be substituted with another substituent, provided the antitumor effect is not impaired. Examples of this other substituent include halogen atoms other than a fluorine atom, and C₁₋₆ alkyl groups.

The fluorine-containing amino acid that is included as an active ingredient of the anticancer agent according to the present invention may be a single type of amino acid, or a combination of two or more types.

The fluorine-containing amino acid that is included as an active ingredient of the anticancer agent according to the present invention can be synthesized by treating an α-amino acid with a fluorinating agent in a state where the carboxyl group and the amino group of the α-amino acid have been protected. The protection of the carboxyl group and amino group and the treatment with a fluorinating agent can be conducted using typical methods. N-fluorobenzenesulfonimide (CAS RN: 133745-75-2) or the like can be used as the fluorinating agent.

Because the anticancer agent according to the present invention has excellent cell membrane permeability, the anticancer agent can be introduced into a target cancer cell simply by bringing the agent into contact with the cancer cell. In those cases where the target cell of the anticancer agent according to the present invention is a cultured cell, the anticancer agent can be introduced into the cell simply by adding the agent to the culture medium and conducting culturing. Further, in the case of animal tissue, for example, by spraying or applying a solution in which the anticancer agent according to the present invention has been dissolved onto the tissue surface, the anticancer agent can be introduced into the cells that constitute the tissue.

The anticancer agent according to the present invention may also contain a pharmacologically acceptable salt of the fluorine-containing amino acid as an active ingredient. Examples of pharmacologically acceptable salts include salts formed with a base such as an inorganic base or an organic base, and salts formed with an acid such as an inorganic acid or an organic acid. Examples of the inorganic bases include alkali metals such as sodium and potassium, alkaline earth metals such as calcium and magnesium, and aluminum and ammonium. Examples of the organic bases include primary amines such as ethanolamine, secondary amines such as diethylamine and diethanolamine, and tertiary amines such as trimethylamine, triethylamine and triethanolamine. Examples of the inorganic acids include hydrochloric acid, phosphoric acid, nitric acid and sulfuric acid. Examples of the organic acids include acetic acid, citric acid, lactic acid, malic acid, maleic acid, benzoic acid, methanesulfonic acid, ethanesulfonic acid, benzenesulfonic acid and p-toluenesulfonic acid.

The anticancer agent according to the present invention is particularly useful as a therapeutic agent for preventing or treating cancer. Examples of the cancer include adrenocortical cancer, anal cancer, bile duct cancer, bladder cancer, breast cancer, cervical cancer, chronic lymphocytic leukemia, chronic myeloid leukemia, colon cancer, endometrial cancer, esophageal cancer, Ewing sarcoma, gallbladder cancer, Hodgkin's disease, hypopharyngeal cancer, laryngeal cancer, oral cancer, liver cancer, non-small cell lung cancer, non-Hodgkin lymphoma, melanoma, mesothelioma, multiple myeloma, ovarian cancer, pancreatic cancer, prostate cancer, stomach cancer, testicular cancer, and thyroid cancer.

By using the anticancer agent according to the present invention as a pharmaceutical composition, either by itself or in a mixture with a pharmacologically acceptable carrier or the like, the anticancer agent can be used for the prevention or treatment of cancer and other diseases in mammals.

Any of the variety of common organic or inorganic carrier substances used as pharmaceutical preparation materials may be used as the abovementioned pharmacologically acceptable carrier, and examples of materials that may be added include excipients, lubricants, binders and disintegrants used in solid preparations; and solvents, solubilizers, suspension agents, isotonicity agents, buffers and analgesics used in liquid preparations. Further, if necessary, other preparation additives such as preservatives, antioxidants, colorants and sweeteners may also be used.

Preferred examples of the excipients include lactose, white sugar, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous silicic acid, synthetic aluminum silicate, and magnesium metasilicate aluminate.

Preferred examples of the lubricants include magnesium stearate, calcium stearate, talc, and colloidal silica.

Preferred examples of the binders include pregelatinized starch, sucrose, gelatin, gum arabic, methyl cellulose, carboxymethyl cellulose, sodium carboxymethyl cellulose, crystalline cellulose, white sugar, D-mannitol, trehalose, dextrin, pullulan, hydroxypropyl cellulose, hydroxypropyl methylcellulose, and polyvinylpyrrolidone.

Preferred examples of the disintegrants include lactose, white sugar, starch, carboxymethyl cellulose, calcium carboxymethyl cellulose, croscarmellose sodium, sodium carboxymethyl starch, light anhydrous silicic acid, and low-substituted hydroxypropyl cellulose.

Preferred examples of the solvents include injection water, physiological saline solution, Ringer's solution, alcohol, propylene glycol, polyethylene glycol, sesame oil, corn oil, olive oil, and cottonseed oil.

Preferred examples of the solubilizers include polyethylene glycol, propylene glycol, D-mannitol, trehalose, benzyl benzoate, ethanol, tris-aminomethane, cholesterol, triethanolamine, sodium carbonate, sodium citrate, sodium salicylate, and sodium acetate.

Preferred examples of the suspension agents include surfactants such as stearyltriethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, sodium carboxymethyl cellulose, methyl cellulose, hydroxymethyl cellulose, hydroxyethyl cellulose, and hydroxypropyl cellulose; as well as polysorbates and polyoxyethylene hydrogenated castor oil.

Preferred examples of the isotonicity agents include sodium chloride, glycerol, D-mannitol, D-sorbitol, and glucose.

Preferred examples of the buffers include buffers solutions of phosphate salts, acetate salts, carbonate salts and citrate salts.

Preferred examples of the analgesics include benzyl alcohol and the like.

Preferred examples of the preservatives include paraoxy benzoate esters, chlorobutanol, benzyl alcohol, phenethyl alcohol, dehydroacetic acid, and sorbic acid.

Preferred examples of the antioxidants include sulfite salts and ascorbate salts.

Preferred examples of the colorants include water-soluble edible tar dyes (for example, food dyes such as food red Nos. 2 and 3, food yellow Nos. 4 and 5, and food blue Nos. 1 and 2), water-insoluble lake dyes (for example, aluminum salts of the above water-soluble edible tar dyes), and natural colorants (for example, β-carotene, chlorophyll, and red iron oxide).

Preferred examples of the sweeteners include sodium saccharin, dipotassium glycyrrhizate, aspartame, and stevia.

Examples of the form of the above pharmaceutical composition include oral agents such as tablets (including sugar-coated tablets, film-coated tablets, sublingual tablets, and orally disintegrating tablets), capsules (including soft capsules and microcapsules), granules, powders, lozenges, syrups, emulsions, suspensions, and films (for example, orally disintegrating films); and parenteral agents such as injections (for example, subcutaneous injections, intravenous injections, intramuscular injections, intraperitoneal injections and drips), external preparations (for example, transdermal preparations and ointments), suppositories (for example, rectal suppositories and vaginal suppositories), pellets, nasal preparations, transpulmonary preparations (inhalants), and eye drops. Each of these preparations can be administered safely either orally or parenterally (for example, locally, rectally or intravenously). These preparations may be rapid release preparations or controlled release preparations such as sustained release preparations (for example, sustained release microcapsules).

In the case of an oral agent, if necessary the agent may be coated for the purpose of masking the taste, or improving the enteric properties or persistence. Examples of coating bases that may be used for the coating process include sugar coating bases, water-soluble film coating bases, enteric film coating bases, and sustained release film coating bases.

Sugar coating bases use white sugar, which may be combined with one or more substances selected from among talc, precipitated calcium carbonate, gelatin, gum arabic, pullulan, and carnauba wax and the like.

Examples of the water-soluble film coating bases include cellulose-based polymers such as hydroxypropyl cellulose, hydroxypropyl methylcellulose, hydroxyethyl cellulose, and methyl hydroxyethyl cellulose; synthetic polymers such as polyvinylacetal diethylaminoacetate, aminoalkyl methacrylate copolymer E (Eudragit E (a product name)), and polyvinylpyrrolidone; and polysaccharides such as pullulan.

Examples of the enteric film coating bases include cellulose-based polymers such as hydroxypropyl methylcellulose phthalate, hydroxypropyl methylcellulose acetate succinate, carboxymethyl ethyl cellulose, and cellulose acetate phthalate; acrylic acid-based polymers such as methacrylic acid copolymer L (Eudragit L (a product name)), methacrylic acid copolymer LD (Eudragit L-30D55 (a product name)), and methacrylic acid copolymer S (Eudragit S (a product name)); and natural polymers such as shellac. Examples of the sustained release film coating bases include cellulose-based polymers such as ethyl cellulose; and acrylic acid-based polymers such as aminoalkyl methacrylate copolymer RS (Eudragit RS (a product name)) and ethyl acrylate-methyl methacrylate copolymer suspension (Eudragit NE (a product name)).

A mixture of two or more of the above coating bases mixed together in appropriate portions may also be used. Further, during the coating process, an opaquer such as titanium oxide or ferric oxide.

The pharmaceutical composition containing the anticancer agent according to the present invention can be produced using commonly used methods in the technical field of pharmaceutical preparations, such as those methods disclosed in the Japanese Pharmacopeia. The amount of the anticancer agent according to the present invention within the pharmaceutical composition differs depending on factors such as the administration route, the form of the composition, and the target dosage of the anticancer agent according to the present invention, but is typically within a range from about 0.1 to 100% by mass.

The dosage of the anticancer agent according to the present invention differs depending on the administration target, the administration route, the target disease, and the symptoms and the like, but for example, in those cases where the anticancer agent is administered either orally or parenterally to an adult human patient, a typical dosage of the anticancer agent according to the present invention per single dose is within a range from about 0.01 to 100 mg/kg body weight, and is preferably from 0.1 to 50 mg/kg body weight, and more preferably from 0.5 to 20 mg/kg body weight.

The anticancer agent according to the present invention or the pharmaceutical composition containing the anticancer agent can be used as a standalone therapeutic agent, or may be used in combination with one or more other cancer treatment methods. Such combination treatments may be conducted simultaneously, separately, or across certain time periods.

Examples of other cancer treatment methods include surgical therapies in which cancer cells are physically resected and removed, chemotherapy methods in which a chemotherapy agent containing an anticancer agent other than the anticancer agent according to the present invention as an active ingredient is administered, immunotherapy methods, and radiation therapies. Examples of the chemotherapy agents include alkylating agents, platinum compounds, antimetabolites, topoisomerase inhibitors, antimicrotubule agents, and antibiotics. Further, examples of immunotherapy methods include methods in which immune checkpoint inhibitors are administered.

The term "simultaneously" used in relation to the dosage form means that in that dosage form, the administration of, and/or treatment with, two or more active ingredients is conducted substantially simultaneously; and as a result of that simultaneous administration, the subject is exposed simultaneously to administration of, and/or treatment with, the two or more active ingredients. In the case of a simultaneous administration, the two or more active ingredients may be administered as a fixed dose combination, may be administered as an equivalent non-fixed dose combination (for example, when two or more different pharmaceutical compositions requiring administration are used substantially simultaneously via the same administration route), or may be administered as a non-fixed dose combination using two or more different administration routes. As a result of these administrations, the subject is exposed essentially simultaneously to the administration of, and/or treatment with, two or more active ingredients.

When used in relation to the dosage form, a "fixed dose combination" means the form of dosage describes the administration of a single pharmaceutical composition having two or more active ingredients.

The term "separately" used in relation to the dosage form means that in that dosage form, the administration of, and/or treatment with, two or more active ingredients is conducted at different times. Simultaneous administration leads to a treatment phase in which the subject is exposed simultaneously to the administration of, and/or treatment with, the two or more active ingredients (for example, within at least one hour, at least 6 hours, or especially within at least 12 hours), whereas separate administration may lead to a treatment phase in which the subject is exposed to the administration of, and/or treatment with, only one of the two or more active ingredients over a certain time period (for example, at least 12 hours, or especially at least one day). Separate administration particularly refers to the case where at least one administration of, and/or treatment with, an active ingredient is conducted at a periodic cycle that is substantially different from daily administration (such as once or twice per day). For example, the administration and/or treatment with one active ingredient may be conducted once or twice per day, with a separate active ingredient being administered every second day, or once per week or an even longer interval.

Administration "across a certain time period" means consecutive administration of, and/or treatment with, two or more active ingredients at different times. In particular, this expression is used to describe an administration method in which following completion of the administration and/or treatment with one active ingredient, administration of one or more other active ingredients is started. In such cases, the administration and/or treatment with one active ingredient can be conducted for several months, and the administration and/or treatment with the other active ingredient then started following completion of the administration of the first active ingredient.

### Examples

The present invention is described further below using a series of examples, but the present invention is not limited to the following examples.

### (Example 1)

Fluorine-containing amino acids in which one hydrogen atom bonded to a carbon atom on the side chain of a branched-chain α-amino acid had been substituted with a fluorine atom were investigated for their effect against cancer cell proliferation. The fluorine-containing amino acids used were monofluoroleucine, trifluoroleucine, hexafluoroleucine (CAS RN: 1214114-26-7, 2-amino-5,5,5-trifluoro-4-(trifluoromethyl)pentanoic acid hydrochloride), monofluorovaline, trifluorovaline (CAS RN: 16063-79-9, 2-amino-3-methyl-4,4,4-trifluorobutanoic acid), and hexafluorovaline. Each of these amino acids was obtained from Namiki Shoji Co., Ltd.

HeLa cells and PANC-1 cells were used as the cancer cells. Both these cells were obtained from ATCC. Further, NHDF cells obtained from Lonza Group Ltd. were used as control non-cancer cells. The culture mediums used were Dulbecco's Modified Eagle Medium (D-MEM, manufactured by FUJIFILM Wako Pure Chemical Corporation) to which 10% FBS had been added (hereinafter also referred to as the "normal medium"), a medium prepared by removing leucine from D-MEM and adding 10% FBS (hereinafter also referred to as the "Leu-removed medium"), and a medium prepared by removing valine from D-MEM and adding 10% FBS (hereinafter also referred to as the "Val-removed medium").

The culture cells were inoculated into a 96-well culture plate at a concentration of 3 × 10³ cells/well, and following adhesion, were cultured for 24, 48 or 72 hours in the normal medium, the Leu-removed medium or Val-removed medium, or a medium prepared by adding the fluorine-containing amino acid to the Leu-removed medium or Val-removed medium in an amount sufficient to achieve a final concentration of 0.8 mM. Following culturing, the cell proliferation rate was measured by SRB assay. As a control, the cell proliferation rate was measured in a similar manner for cells having a culture time of 0 hours.

The SRB assays were conducted in the following manner. Following cell culturing, the medium was removed, and 10% (wt/vol) of trichloroacetic acid was added to fix the cells. Subsequently, the fixed cells were washed three times with tap water, and then stained by adding a solution containing 0.4% (wt/vol) of sulforhodamine B dissolved in 1% acetic acid. Following subsequent washing three times with a 1% acetic acid solution, a 10 mM Tris solution was added, and the light absorbance at 540 nm was measured. The 540 mm absorbance value for each sample was used to indicate the cell quantity, and for each sample, the relative cell proliferation rate (%) at a culture time of X hours was determined using the formula below. [relative cell proliferation rate (%) at culture time X hours] = [540 nm absorbance of sample at culture time X hours] / [540 nm absorbance of sample at culture time 0 hours] × 100 (%)

The results for the HeLa cells to which fluoroleucine had been added are shown in Tables 1 to 3, the results for the PANC-1 cells to which fluoroleucine had been added are shown in Tables 4 to 6, the results for the NHDF cells to which fluoroleucine had been added are shown in Tables 7 to 9, the results for the HeLa cells to which fluorovaline had been added are shown in Tables 10 to 12, the results for the PANC-1 cells to which fluorovaline had been added are shown in Tables 13 to 15, and the results for the NHDF cells to which fluorovaline had been added are shown in Tables 16 to 18. In the tables, "DMEM + 10% FBS" indicates the relative cell proliferation rate for cells cultured in the normal medium, "DMEM-Leu + 10% FBS" indicates the relative cell proliferation rate for cells cultured in the Leu-removed medium, "DMEM-Leu + Leu(1F) + 10% FBS", "DMEM-Leu + Leu(3F) + 10% FBS" and "DMEM-Leu + Leu(6F) + 10% FBS" indicate the relative cell proliferation rates for cells cultured in a Leu-removed medium to which monofluoroleucine, trifluoroleucine and hexafluoroleucine respectively had been added. "DMEM-Val + 10% FBS" indicates the relative cell proliferation rate for cells cultured in the Val-removed medium, and "DMEM-Val + Val(1F) + 10% FBS", "DMEM-Val + Val(3F) + 10% FBS" and "DMEM-Val + Val(6F) + 10% FBS" indicate the relative cell proliferation rates for cells cultured in a Val-removed medium to which monofluorovaline, trifluorovaline and hexafluorovaline respectively had been added.

**[Table 1]**

| | Relative cell proliferation rate of HeLa cells [%] | | |
|---|---|---|---|
| Culture time [h] | DMEM +10%FBS | DMEM-Leu +10%FBS | DMEM-Leu +Leu(1F) +10%FBS |
| 0 | 100 | 100 | 100 |
| 24 | 147.9 | 119.6 | 82.3 |
| 48 | 347.6 | 265.6 | 90.6 |
| 72 | 614.0 | 584.4 | 82.0 |

**[Table 2]**

| | Relative cell proliferation rate of HeLa cells [%] | | |
|---|---|---|---|
| Culture time [h] | DMEM +10%FBS | DMEM-Leu +10%FBS | DMEM-Leu +Leu(3F) +10%FBS |
| 0 | 100 | 100 | 100 |
| 24 | 127.2 | 119.6 | 78.5 |
| 48 | 267.7 | 265.6 | 91.6 |
| 72 | 570.7 | 584.4 | 89.5 |

**[Table 3]**

| | Relative cell proliferation rate of HeLa cells [%] | | |
|---|---|---|---|
| Culture time [h] | DMEM +10%FBS | DMEM-Leu +10%FBS | DMEM-Leu +Leu(6F) +10%FBS |
| 0 | 100 | 100 | 100 |
| 24 | 145.9 | 151.0 | 137.3 |
| 48 | 296.8 | 295.4 | 264.0 |
| 72 | 520.1 | 455.3 | 472.5 |

**[Table 4]**

| | Relative cell proliferation rate of PANC-1 cells [%] | | |
|---|---|---|---|
| Culture time [h] | DMEM +10%FBS | DMEM-Leu +10%FBS | DMEM-Leu +Leu(1F) +10%FBS |
| 0 | 100 | 100 | 100 |
| 24 | 110.1 | 109.2 | 67.6 |
| 48 | 205.0 | 172.0 | 47.9 |
| 72 | 330.5 | 232.2 | 40.9 |

**[Table 5]**

| | Relative cell proliferation rate of PANC-1 cells [%] | | |
|---|---|---|---|
| Culture time [h] | DMEM +10%FBS | DMEM-Leu +10%FBS | DMEM-Leu +Leu(3F) +10%FBS |
| 0 | 100 | 100 | 100 |
| 24 | 110.1 | 109.2 | 77.1 |
| 48 | 205.0 | 172.0 | 87.0 |
| 72 | 330.5 | 232.2 | 75.5 |

**[Table 6]**

| | Relative cell proliferation rate of PANC-1 cells [%] | | |
|---|---|---|---|
| Culture time [h] | DMEM +10%FBS | DMEM-Leu +10%FBS | DMEM-Leu +Leu(6F) +10%FBS |
| 0 | 100 | 100 | 100 |
| 24 | 109.2 | 110.1 | 75.5 |
| 48 | 172.0 | 205.0 | 96.5 |
| 72 | 232.2 | 330.5 | 193.6 |

**[Table 7]**

| | Relative cell proliferation rate of NHDF cells [%] | | |
|---|---|---|---|
| Culture time [h] | DMEM +10%FBS | DMEM-Leu +10%FBS | DMEM-Leu +Leu(1F) +10%FBS |
| 0 | 100 | 100 | 100 |
| 24 | 109.1 | 108.2 | 92.0 |
| 48 | 128.6 | 103.1 | 66.4 |
| 72 | 214.9 | 197.9 | 87.7 |

**[Table 8]**

| | Relative cell proliferation rate of NHDF cells [%] | | |
|---|---|---|---|
| Culture time [h] | DMEM +10%FBS | DMEM-Leu +10%FBS | DMEM-Leu +Leu(3F) +10%FBS |
| 0 | 100 | 100 | 100 |
| 24 | 160.8 | 155.8 | 138.5 |
| 48 | 212.0 | 202.9 | 138.2 |
| 72 | 302.9 | 286.0 | 185.6 |

**[Table 9]**

| | Relative cell proliferation rate of NHDF cells [%] | | |
|---|---|---|---|
| Culture time [h] | DMEM +10%FBS | DMEM-Leu +10%FBS | DMEM-Leu +Leu(6F) +10%FBS |
| 0 | 100 | 100 | 100 |
| 24 | 100.2 | 110.6 | 104.7 |
| 48 | 150.4 | 166.7 | 130.1 |
| 72 | 213.9 | 205.7 | 179.1 |

**[Table 10]**

| | Relative cell proliferation rate of HeLa cells [%] | | |
|---|---|---|---|
| Culture time [h] | DMEM +10%FBS | DMEM-Val +10%FBS | DMEM-Val+Val(1F) +10%FBS |
| 0 | 100 | 100 | 100 |
| 24 | 120.6 | 122.7 | 72.83 |
| 48 | 306.5 | 338.8 | 118.0 |
| 72 | 706.8 | 652.5 | 273.6 |

**[Table 11]**

| | Relative cell proliferation rate of HeLa cells [%] | | |
|---|---|---|---|
| Culture time [h] | DMEM +10%FBS | DMEM-Val +10%FBS | DMEM-Val +Val(3F) +10%FBS |
| 0 | 100 | 100 | 100 |
| 24 | 145.5 | 143.5 | 151.7 |
| 48 | 280.9 | 278.1 | 287.6 |
| 72 | 510.2 | 489.9 | 494.3 |

**[Table 12]**

| | Relative cell proliferation rate of HeLa cells [%] | | |
|---|---|---|---|
| Culture time [h] | DMEM +10%FBS | DMEM-Val +10%FBS | DMEM-Val +Val(6F) +10%FBS |
| 0 | 100 | 100 | 100 |
| 24 | 127.2 | 125.8 | 88.1 |
| 48 | 267.7 | 255.9 | 93.7 |
| 72 | 570.7 | 589.8 | 67.6 |

**[Table 13]**

| | Relative cell proliferation rate of PANC-1 cells [%] | | |
|---|---|---|---|
| Culture time [h] | DMEM +10%FBS | DMEM-Val +10%FBS | DMEM-Val +Val(1F) +10%FBS |
| 0 | 100 | 100 | 100 |
| 24 | 134.8 | 123.7 | 88.8 |
| 48 | 308.3 | 186.5 | 103.8 |
| 72 | 430.1 | 260.5 | 105.1 |

**[Table 14]**

| | Relative cell proliferation rate of PANC-1 cells [%] | | |
|---|---|---|---|
| Culture time [h] | DMEM +10%FBS | DMEM-Val +10%FBS | DMEM-Val +Val(3F) +10%FBS |
| 0 | 100 | 100 | 100 |
| 24 | 134.8 | 123.7 | 108.9 |
| 48 | 308.3 | 186.5 | 161.2 |
| 72 | 430.1 | 260.5 | 253.7 |

**[Table 15]**

| | Relative cell proliferation rate of PANC-1 cells [%] | | |
|---|---|---|---|
| Culture time [h] | DMEM +10%FBS | DMEM-Val +10%FBS | DMEM-Val +Val(6F) +10%FBS |
| 0 | 100 | 100 | 100 |
| 24 | 134.8 | 123.7 | 104.3 |
| 48 | 308.3 | 186.5 | 133.4 |
| 72 | 430.1 | 260.5 | 141.3 |

**[Table 16]**

| | Relative cell proliferation rate of NHDF cells [%] | | |
|---|---|---|---|
| Culture time [h] | DMEM +10%FBS | DMEM-Val +10%FBS | DMEM-Val +Val(1F) +10%FBS |
| 0 | 100 | 100 | 100 |
| 24 | 109.1 | 89.2 | 91.5 |
| 48 | 128.6 | 76.7 | 70.4 |
| 72 | 214.9 | 149.5 | 110.5 |

**[Table 17]**

| | Relative cell proliferation rate of NHDF cells [%] | | |
|---|---|---|---|
| Culture time [h] | DMEM +10%FBS | DMEM-Val +10%FBS | DMEM-Val +Val(3F) +10%FBS |
| 0 | 100 | 100 | 100 |
| 24 | 108.2 | 91.5 | 83.0 |
| 48 | 152.4 | 110.9 | 104.6 |
| 72 | 206.5 | 140.2 | 116.6 |

**[Table 18]**

| | Relative cell proliferation rate of NHDF cells [%] | | |
|---|---|---|---|
| Culture time [h] | DMEM +10%FBS | DMEM-Val +10%FBS | DMEM-Val +Val(6F) +10%FBS |
| 0 | 100 | 100 | 100 |
| 24 | 160.8 | 128.4 | 109.8 |
| 48 | 212.0 | 129.4 | 104.4 |
| 72 | 302.9 | 167.0 | 125.2 |

As illustrated in Tables 1 to 6, with the HeLa cells and the PANC-1 cells, which are both cancer cell-derived cultured lines, in the case of the mediums prepared by adding monofluoroleucine or trifluoroleucine to the Leu-removed medium, the relative cell proliferation rate after 72 hours of culture was less than 100%, indicating that cell proliferation was significantly inhibited by monofluoroleucine or trifluoroleucine. In contrast, in the case of the medium containing added hexafluoroleucine, the relative cell proliferation rate after 72 hours of culture was similar to that of the Leu-removed medium in the case of the HeLa cells, and although being slightly lower than the Leu-removed medium in the case of the PANC-1 cells, was still close to 200%, confirming that cell proliferation had not been inhibited. Based on these results, it was evident that monofluoroleucine and trifluoroleucine each exhibit a cell proliferation inhibitory action against cancer cells.

Further, as illustrated in Tables 7 to 9, in the case of the NHDF cells, which represent a non-cancer cell-derived cultured line, the relative cell proliferation rate after 72 hours of culture was less than 100% in the medium to which monofluoroleucine had been added, indicating a cell proliferation inhibitory action, but the effect observed was smaller than that observed for the HeLa cells and PANC-1 cells, whereas in the case of the medium to which trifluoroleucine had been added, although the relative cell proliferation rate was smaller than the Leu-removed medium, the rate was close to 200%, confirming that cell proliferation had not been inhibited. The relative cell proliferation rate after 72 hours of culture in the medium to which hexafluoroleucine had been added was similar to that of the Leu-removed medium, and no cell proliferation inhibitory action was observed. Based on these results, it is thought that trifluoroleucine has a cell proliferation inhibitory action that exhibits a high specificity for cancer cells, making it particularly desirable as an active ingredient for an anticancer agent.

As illustrated in Tables 10 to 15, with the HeLa cells, in the medium prepared by adding hexafluorovaline to the Val-removed medium, the relative cell proliferation rate after 72 hours of culture was less than 100%, indicating that cell proliferation was significantly inhibited. In the medium prepared by adding monofluorovaline to the Val-removed medium, the relative cell proliferation rate after 72 hours of culture was 273.6%, and although cell proliferation had occurred, the rate was clearly lower than the relative cell proliferation rate observed for the Val-removed medium, indicating that cell proliferation had been inhibited. On the other hand, with the PANC-1 cells, in the medium prepared by adding monofluorovaline to the Val-removed medium, the relative cell proliferation rate after 72 hours of culture was about 100%, indicating that cell proliferation was significantly inhibited. In the medium prepared by adding hexafluorovaline to the Val-removed medium, the relative cell proliferation rate after 72 hours of culture was 141.3%, and although cell proliferation had occurred, the rate was clearly lower than the relative cell proliferation rate observed for the Val-removed medium, indicating that cell proliferation had been inhibited. In contrast, in the case of the medium prepared by adding trifluorovaline to the Val-removed medium, with both the HeLa cells and the PANC-1 cells, the relative cell proliferation rates after 72 hours of culture were similar to that of the Val-removed medium, indicating that cell proliferation had not been inhibited. Based on these results, it was evident that monofluorovaline and hexafluorovaline each exhibit a cell proliferation inhibitory action against cancer cells.

Further, as illustrated in Tables 16 to 18, in the case of the NHDF cells, the relative cell proliferation rates after 72 hours of culture in the mediums to which monofluorovaline or hexafluorovaline had been added were lower than the relative cell proliferation rate observed for the Val-removed medium, indicating a cell proliferation inhibitory action, but the effect observed was significantly weaker than the cell proliferation inhibitory action observed for the HeLa cells and PANC-1 cells. Based on these results, it can be stated that although monofluorovaline and hexafluorovaline each exhibit a cell proliferation inhibitory action against both cancer cells and normal cells, the cell proliferation inhibitory action against cancer cells is much stronger than the action against normal cells.

### (Example 2)

Fluorine-containing amino acids in which one hydrogen atom bonded to a carbon atom on the side chain of a branched-chain α-amino acid had been substituted with a fluorine atom were investigated for their anticancer effect tumor tissue in mice. The fluorine-containing amino acids used were trifluoroleucine (CAS RN: 2260931-25-5) (obtained from Namiki Shoji Co., Ltd.), a mixture of 3-fluoroisoleucine (CAS RN: 79205-62-2) and 4-fluoroisoleucine (CAS RN: 2643396-11-4) (hereinafter abbreviated as Ile(3/4F) or sometimes referred to as simply "monofluoroisoleucine"), and 5-fluoro-5-methyl-norleucine (CAS RN: 1361228-49-0). The monofluoroisoleucine and 5-fluoro-5-methyl-norleucine were synthesized in accordance with the method disclosed by Nodwell et al. (Non-Patent Document 4).

### (1) Anticancer Effect of Trifluoroleucine

First, 0.1 mL samples of an HeLa cell solution with a concentration of 1.0 × 10⁷ cells/mL were transplanted beneath the skin of SCID/SCID (immunodeficient) mice. Starting 7 days after the HeLa cell transplantation, PBS (control group), leucine (5 mg/kg mouse bodyweight) or trifluoroleucine (5 mg/kg mouse bodyweight) was administered intraperitoneally every day for two weeks. The growth of the tumor tissue was observed over time from the time of HeLa transplantation, and the tumor volume (mm³) was calculated. The tumor volume (mm³) was calculated from the major diameter (mm) of the tumor tissue and the minor diameter (mm) of the tumor tissue using the formula shown below. [Tumor volume (mm3)] = [tumor major diameter (mm)]2 × [tumor minor diameter (mm)] / 2

**[Table 19]**

| Time since HeLa cell transplantation [days] | Tumor volume [mm³] | | |
|---|---|---|---|
| | control(PBS) | Leu | Leu(3F) |
| 7 | 113.1 | 77.4 | 135.6 |
| 10 | 212.1 | 190.1 | 169.4 |
| 12 | 318.3 | 355.3 | 204.1 |
| 14 | 436.3 | 385.1 | 200.6 |
| 17 | 702.8 | 714.2 | 315.3 |
| 19 | 1279.8 | 1094.6 | 460.2 |
| 21 | 2029.4 | 1536.5 | 704.0 |

The measurement results for tumor volume are listed in Table 19. In the table, "Leu" and "Leu(3F)" indicate the results for the mice administered with leucine or trifluoroleucine respectively. In the mice in the trifluoroleucine administration group, increase in the size of the tumor tissue was dramatically inhibited compared with the control group and the leucine administration group. These results confirmed that trifluoroleucine also has an in vivo cancer cell proliferation inhibitory action.

### (2) Anticancer Effect of Monofluoroisoleucine

With the exceptions of using monofluoroisoleucine or 5-fluoro-5-methyl-norleucine (CAS RN: 1361228-49-0) instead of the trifluoroleucine, and starting the administration of the PBS, 5-fluoro-5-methyl-norleucine or monofluoroisoleucine 11 days after rather than 7 days after the HeLa cell transplantation, the same procedure as (1) above was used to measure the volume (mm³) of the tumor formed by the subcutaneously transplanted HeLa cells over time.

**[Table 20]**

| Time since HeLa cell transplantation [days] | Tumor volume [mm³] | | |
|---|---|---|---|
| | control(PBS) | 5F-NorLeu | Ile(3F/4F) |
| 11 | 208.8 | 230.6 | 193.3 |
| 13 | 364.7 | 373.9 | 316.2 |
| 15 | 724.7 | 684.9 | 583.3 |
| 18 | 1194.4 | 823.5 | 799.5 |
| 20 | 1673.3 | 1291.7 | 1075.2 |
| 22 | 2363.9 | 1951.5 | 1407.2 |

The measurement results for tumor volume are listed in Table 20. In the table, "5F-NorLeu" and "Ile(3F/4F)" indicate the results for the mice administered with 5-fluoro-5-methyl-norleucine or monofluoroisoleucine respectively. In the mice in the monofluoroisoleucine administration group, increase in the size of the tumor tissue was dramatically inhibited compared with the control group and the 5-fluoro-5-methyl-norleucine administration group. These results confirmed that monofluoroisoleucine also has an in vivo cancer cell proliferation inhibitory action.

### (Example 3)

Monofluoroglutamine (Gln(1F)) in which one hydrogen atom bonded to a carbon atom on the side chain of the linear-chain α-amino acid glutamine (Gln) had been substituted with a fluorine atom was investigated for its effect against cancer cell proliferation. The monofluoroglutamine isomers (2S,4S)Gln(1F) and (2S,4R)Gln(1F) were used. These compounds were both obtained from Namiki Shoji Co., Ltd.

### (1) Anticancer Effect in Tumor-Bearing Mice

First, 0.1 mL samples of an HeLa cell solution with a concentration of 1.0 × 10⁷ cells/mL were transplanted beneath the skin of SCID/SCID (immunodeficient) mice. Starting 7 days after the HeLa cell transplantation, PBS (control group), glutamine (Gln) (5 mg/kg mouse bodyweight), (2S,4S)Gln(1F) (5 mg/kg mouse bodyweight) or (2S,4R)Gln(1F) (5 mg/kg mouse bodyweight) was administered intraperitoneally every day for two weeks. The growth of the tumor tissue was observed over time from the time of HeLa cell transplantation, and the tumor volume (mm³) was calculated. The tumor volume (mm³) was determined in the same manner as that described in Example 2.

**[Table 21]**

| Time since HeLa cell transplantation [days] | Tumor volume [mm³] | | | |
|---|---|---|---|---|
| | control(PBS) | Gln | (2S,4S)-Gln(1F) | (2S,4R)-Gln(1F) |
| 7 | 70.5 | 76.0 | 72.1 | 74.6 |
| 9 | 113.7 | 134.7 | 96.9 | 101.6 |
| 11 | 184.7 | 215.4 | 149.0 | 147.2 |
| 14 | 354.0 | 488.0 | 207.1 | 219.1 |
| 16 | 575.9 | 734.1 | 242.3 | 287.0 |
| 18 | 741.9 | 939.6 | 357.2 | 307.7 |
| 21 | 995.4 | 1256.0 | 439.9 | 431.4 |

The measurement results for tumor volume are listed in Table 21. In the Gln administration group, the tumor volume was even larger than the control group. In contrast, in both of the monofluoroglutamine administration groups (the (2S,4S)Gln(1F) administration group and the (2S,4R)Gln(1F) administration group), increase in the size of the tumor tissue was dramatically inhibited compared with the control group. These results confirmed that monofluoroglutamine has an in vivo cancer cell proliferation inhibitory action.

### (2) SRB Assay

SRB assays were conducted in the same manner as Example 1 with the exceptions of using a medium prepared by removing glutamine from D-MEM and adding 10% FBS (hereinafter also referred to as the "Gln-removed medium") as the culture medium instead of the Leu-removed medium, using monofluoroglutamine ((2S,4S)Gln(1F) or (2S,4R)Gln(1F)) as the fluorine-containing amino acid, and altering the amount of monofluoroglutamine added to the Gln-removed medium to an amount sufficient to achieve a final concentration of 4 mM.

The relative cell proliferation rates (%) measured in the SRB assays (relative to a value of 100% for the number of cells at a culture time of 0 hours) are shown in Tables 22 to 24.

**[Table 22]**

| | Relative cell proliferation rate of HeLa cells [%] | | | |
|---|---|---|---|---|
| Culture time [h] | DMEM +10%FBS | DMEM-Gln +10%FBS | DMEM-Gln +(2S,4S)Gln(1F) +10%FBS | DMEM-Gln +(2S,4R)Gln(1F) +10%FBS |
| 0 | 100 | 100 | 100 | 100 |
| 24 | 129.8 | 102.9 | 29.2 | 46.8 |
| 48 | 393.6 | 176.7 | 32.1 | 21.5 |
| 72 | 710.3 | 237.1 | 25.0 | 23.1 |

**[Table 23]**

| | Relative cell proliferation rate of PANC-1 cells [%] | | | |
|---|---|---|---|---|
| Culture time [h] | DMEM +10%FBS | DMEM-Gln +10%FBS | DMEM-Gln +(2S,4S)Gln(1F) +10%FBS | DMEM-Gln +(2S,4R)Gln(1F) +10%FBS |
| 0 | 100 | 100 | 100 | 100 |
| 24 | 152.2 | 71.4 | 31.5 | 65.0 |
| 48 | 382.0 | 112.9 | 27.3 | 34.7 |
| 72 | 925.1 | 134.5 | 25.7 | 25.7 |

**[Table 24]**

| | Relative cell proliferation rate of NHDF cells [%] | | | |
|---|---|---|---|---|
| Culture time [h] | DMEM +10%FBS | DMEM-Gln +10%FBS | DMEM-Gln +(2S,4S)Gln(1F) +10%FBS | DMEM-Gln +(2S,4R)Gln(1F) +10%FBS |
| 0 | 100 | 100 | 100 | 100 |
| 24 | 197.4 | 191.5 | 157.4 | 132.5 |
| 48 | 250.9 | 231.6 | 129.4 | 46.5 |
| 72 | 246.7 | 210.2 | 82.9 | 26.3 |

As illustrated in Tables 22 and 23, with the HeLa cells and the PANC-1 cells, which are both cancer cell-derived cultured lines, the relative cell proliferation rate after 24 hours of culture was less than 100% in the medium prepared by adding monofluoroglutamine to the Gln-removed medium, indicating that cell proliferation was significantly inhibited by monofluoroglutamine. Further, as illustrated in Table 24, in the case of the NHDF cells, which represent a non-cancer cell-derived cultured line, although a cell proliferation inhibitory action due to the monofluoroglutamine addition was observed, that inhibitory effect was smaller than that observed for the cancer cell-derived cultured lines. In particular, in the case of (2S,4S)Gln(1F), the specificity of the cell proliferation inhibitory action for cancer cells was higher than that observed for (2S,4R)Gln(1F), making it particularly desirable as an active ingredient for an anticancer agent.

### (Example 4)

The effect of difluoroglutamine (di-F-Gln(F)) against cancer cell proliferation was investigated.

### (1) Anticancer Effect in Tumor-Bearing Mice

First, 0.1 mL samples of an HeLa cell solution with a concentration of 1.0 × 10⁷ cells/mL were transplanted beneath the skin of SCID/SCID (immunodeficient) mice. Starting 7 days after the HeLa cell transplantation, PBS (control group), glutamine (Gln) (5 mg/kg mouse bodyweight), or di-F-Gln(F) (5 mg/kg mouse bodyweight) was administered intraperitoneally every day for two weeks. The growth of the tumor tissue was observed over time from the time of HeLa transplantation, and the tumor volume (mm³) was calculated. The tumor volume (mm³) was determined in the same manner as that described in Example 2.

**[Table 25]**

| Time since HeLa cell transplantation [days] | Tumor volume [mm³] | | |
|---|---|---|---|
| | control(PBS) | Gln | di-F-Gln |
| 7 | 94.8 | 97.3 | 99.3 |
| 9 | 184.1 | 259.1 | 144.5 |
| 11 | 258.2 | 341.0 | 179.7 |
| 14 | 475.0 | 721.9 | 246.5 |
| 16 | 712.9 | 1094.6 | 448.2 |
| 18 | 933.6 | 1348.3 | 480.8 |
| 21 | 1267.2 | 1849.5 | 645.5 |

The measurement results for tumor volume are listed in Table 25. In the Gln administration group, the tumor volume was even larger than the control group. In contrast, in the difluoroglutamine (di-F-Gln(F) administration group, increase in the size of the tumor tissue was dramatically inhibited compared with the control group. These results confirmed that difluoroglutamine has an in vivo cancer cell proliferation inhibitory action.

### (2) SRB Assay

SRB assays were conducted in the same manner as Example 1 with the exceptions of using the Gln-removed medium as the culture medium instead of the Leu-removed medium, using difluoroglutamine as the fluorine-containing amino acid, and altering the amount of difluoroglutamine added to the Gln-removed medium to an amount sufficient to achieve a final concentration of 4 mM.

The relative cell proliferation rates (%) measured in the SRB assays (relative to a value of 100% for the number of cells at a culture time of 0 hours) are shown in Tables 26 and 27. The results for the HeLa cells to which difluoroglutamine had been added are shown in Table 26, and the results for the PANC-1 cells to which difluoroglutamine had been added are shown in Table 27. In the tables, "DMEM + 10% FBS" indicates the relative cell proliferation rate for cells cultured in the normal medium, "DMEM-Gln + 10% FBS" indicates the relative cell proliferation rate for cells cultured in the Gln-removed medium, and "DMEM-Gln + di-F-Gln + 10% FBS" indicates the relative cell proliferation rate for cells cultured in a medium prepared by adding difluoroglutamine to the Gln-removed medium.

**[Table 26]**

| | Relative cell proliferation rate of HeLa cells [%] | | |
|---|---|---|---|
| Culture time [h] | DMEM +10%FBS | DMEM-Gln +10%FBS | DMEM-Gln + di-F-Gln +10%FBS |
| 0 | 100 | 100 | 100 |
| 24 | 219.9 | 129.6 | 98.8 |
| 48 | 338.8 | 260.0 | 43.7 |
| 72 | 673.6 | 297.4 | 52.4 |

**[Table 27]**

| | Relative cell proliferation rate of PANC-1 cells [%] | | |
|---|---|---|---|
| Culture time [h] | DMEM +10%FBS | DMEM-Gln +10%FBS | DMEM-Gln + di-F-Gln +10%FBS |
| 0 | 100 | 100 | 100 |
| 24 | 203.9 | 138.7 | 74.8 |
| 48 | 369.5 | 173.8 | 57.7 |
| 72 | 611.2 | 208.3 | 26.0 |

As illustrated in Tables 26 and 27, with the HeLa cells and the PANC-1 cells, which are both cancer cell-derived cultured lines, in the case of the medium prepared by adding difluoroglutamine to the Gln-removed medium, the relative cell proliferation rate after 72 hours of culture was less than 100%, indicating that cell proliferation was significantly inhibited by difluoroglutamine. Based on these results, it was evident that difluoroglutamine exhibits a cell proliferation inhibitory action against cancer cells.

## Claims

1. An anticancer agent comprising, as an active ingredient, a fluorine-containing amino acid in which at least one hydrogen atom bonded to a carbon atom of an α-amino acid has been substituted with a fluorine atom, or a pharmacologically acceptable salt thereof.

2. The anticancer agent according to Claim 1, wherein the fluorine-containing amino acid has a monofluoromethyl group, a difluoromethyl group, or a trifluoromethyl group.

3. The anticancer agent according to Claim 1, wherein the α-amino acid is at least one amino acid selected from the group consisting of valine, leucine, isoleucine and glutamine.

4. The anticancer agent according to Claim 1, wherein the fluorine-containing amino acid is at least one amino acid selected from the group consisting of monofluoroleucine, trifluoroleucine, monofluorovaline, hexafluorovaline, monofluoroisoleucine, monofluoroglutamine and difluoroglutamine.

5. A pharmaceutical composition comprising the anticancer agent according to any one of Claims 1 to 4.
